(19) Europäisches Patentamt
European Patent Office
Office européen des brevets

(11) Veröffentlichungsnummer: **0 099 057**
**B1**

(12) **EUROPÄISCHE PATENTSCHRIFT**

(45) Veröffentlichungstag der Patentschrift: **14.10.87**

(21) Anmeldenummer: **83106587.5**

(22) Anmeldetag: **06.07.83**

(51) Int. Cl.[4]: **C 07 K 5/06,** C 07 K 5/08, C 07 K 1/02, C 07 K 1/06, A 61 K 37/02

(54) **N-Acylderivate von Peptiden, ihre Herstellung und Verwendung bei der Bekämpfung von Krankheiten und Mittel dafür.**

(30) Priorität: **14.07.82 DE 3226241**

(43) Veröffentlichungstag der Anmeldung: **25.01.84 Patentblatt 84/4**

(45) Bekanntmachung des Hinweises auf die Patenterteilung: **14.10.87 Patentblatt 87/42**

(84) Benannte Vertragsstaaten: **AT BE CH DE FR GB IT LI NL SE**

(56) Entgegenhaltungen:
**EP-A-0 074 787**
**WO-A-80/00216**
**FR-A-2 373 517**
**US-A-3 795 738**
**US-A-4 018 912**
**US-A-4 278 595**

**CHEMICAL ABSTRACTS, Band 94, Nr. 11, 16. März 1981, Seite 765, Nr. 84499w, Columbus, Ohio, USA, R. URBAN et al.: "The synthesis of alamethicin-fragments by four component condensation"**

(73) Patentinhaber: **BASF Aktiengesellschaft, Carl-Bosch- Strasse 38, D-6700 Ludwigshafen (DE)**

(72) Erfinder: **Albrecht, Hans Peter, Dr., Brombeerweg 5, D-6940 Weinheim (DE)**
Erfinder: **Kreiskott, Horst, Dr., Am Boehlig, D-6706 Wachenheim (DE)**

**EP 0 099 057 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1987

**Beschreibung**

Die Erfindung betrifft neue N-Acylderivate von Peptiden, Verfahren zu deren Herstellung, Arzneimittel, die diese neuen Verbindungen enthalten, sowie deren Verwendung bei der Bekämpfung von Krankheiten.

Das Tripeptid L-Pro-L-Leu-Gly-$NH_2$ (MIF) ist der Hemmfaktor des Melanocyten-stimulietenden Hormons (The Merck-Index, 9. Aufl. 1976). Neben seiner endokrinen Wirkung übt das Tripeptid im Zentralnervensystem einen Neurotransmitter oder Neuromodulator-Effekt aus. Klinische Studien zeigten, daß MIF allein oder in Kombination mit L-Dopa bei Parkinson-Patienten Tremor, Rigor und Akinese günstig beeinflußt (A.J. Kastin u. A. Barbeau Can. Med. Asoc. J. 107, 1097 (1972) und F. Gerstenbrand et al, Wien. Klin. Wschr. 87, 822 (1975)).

Einer breiten therapeutischen Anwendung dieses Tripeptids steht jedoch die ungenügende orale Wirksamkeit und die kurze Wirkdauer im Wege.

Es ist weiter versucht worden - unter Erhaltung der pharmakologischen Wirksamkeit von H-Pro-Leu-Gly-$NH_2$ - durch Molekülabwandlung zu Verbindungen mit oraler Wirksamkeit und ausreichender Wirkdauer zu kommen.

Die Bemühungen konzentrierten sich auf den Austausch des mittelständigen L-Leucins gegen D-Leucin (US-PS 4 278 595) oder gegen N-Alkylderivate von L- und D-Leucin (DE-OS 26 33 976).

Der Ersatz des L-Prolins durch Säuren oder Aminosäuren wurde bisher nur wenig untersucht, da er, abgesehen vom Austausch gegen L-Pyroglutaminsäure, der unschädlich ist (S. Björkman et al, Act. Pharm. Suec. 13, 289 (1976)) nur zu pharmakologisch inaktiven Produkten geführt hat (R.C. Johnson et al, J. Med. Chem. 21, 165 (1978).

Es wurde nun gefunden, daß N-Acylderivate von Peptiden der Formel I

$$X\text{-}NH\text{-}CR^2R^3\text{-}CO\text{-}NH\text{-}CH_2\text{-}CO\text{-}R^1 \quad (I),$$

in der

$R^1$, einen $C_{1-5}$-Alkoxyrest oder einen Aminorest der Formel, $NR^4R^5$ darstellt, worin $R^4$ und $R^5$ gleich oder verschieden sind und Wasserstoff oder $C_{1-5}$-Alkylreste dedeuten,

$R^2$ und $R^3$ gleich oder verschieden sind und $C_{1-5}$-Alkyl-reste oder Phenyl-, Benzyl- oder Naphthylreste oder zusammen mit dem ihnen gemeinsamen C-Atom auch ein aliphatisches Ringsystem mit 3 bis 7 Kohlenstoffatomen oder einen 1,1- oder 2,2-Indandiylrest bedeuten und

X einen Pyrrol-2-ylcarbonyl-, Indol-2-ylcarbonyl-, Cyclopentylcarbonyl-, L-Pyrrolidin-2-ylcarbonyl-, L-4,5-Dehydropyrrolidin-2-ylcarbonyl-, L-5-Cxopyrrolidin-2yl-carbonyl-, L-1,3-Thiazolidin-4-ylcarbonyl- oder L-1,4-Thiazan-2-ylcarbonylrest bedeutet

sowie deren Salze mit physiologisch verträglichen Säuren sehr gute Wirkungen auf das Zentralnervensystem zeigen.

In der Formel I bedeutet $R^1$ vorzugsweise den Rest $NR^4R^5$.

Zur Salzbildung eignen sich insbesondere folgende physiologisch verträglichen Säuren: Salzsäure, Schwefelsäure, Phosphorsäure, Essigsäure, Milchsäure, Bernsteinsäure, Fumarsäure und Äpfelsaure.

Falls $R^2$ und $R^3$ voneinander verschieden sind, liegen die Ausgangsstoffe der Formel $H_2N$-$CR^2R^3$-COOH als Racemate vor. Sie können nach bekannten Methoden in ihre Antipoden getrennt werden und als reine D- bzw. L-Verbindungen in die Reaktion eingesetzt werden.

Sofern X-OH eine optisch aktive Aminosäure darstellt, kann man auch die Ausgangsstoffe der Formel $H_2N$-$CR^2R^3$-COOH in Form ihrer Racemate in die Reaktionsfolge einsetzen. Man erhält dann Gemische zweier diastereomerer Peptide, die sich chromatographisch oder durch Kristallisation trennen lassen.

Die neuen Verbindungen werden hergestellt, indem man die Ausgangssubstanzen X-OH, $H_2N$-$CR^2R^3$-COOH und $H_2N$-$CH_2$-CO-$R^1$, worin X, $R^1$, $R^2$ und $R^3$ die angegebene Bedeutung besitzen, nach den in der Peptidchemie üblichen Verfahren miteinander kondensiert.

Die Synithese kann so durchgeführt werden, daß man zuerst X-OH mit $H_2N$-$CR^2R^3$-COOH kondensiert und dann die so erhaltene Verbindung mit $H_2N$-$CH_2$-CO-$R^1$ umsetzt oder zuerst $H_2N$-$CR^2R^3$-COOH mit $H_2N$-$CH_2$-CO-$R^1$ kondensiert und das dabei entstehende Peptid mit X-OH umsetzt. Die einzelnen Reaktionsstufen werden nach Blockierung der in den einzelnen Stufen nicht beteiligten Amin- und Säurefunktionen durch geeignete Schutzgruppen durchgeführt. Die Abspaltung der Schutzgruppen nach erfolgter Reaktion erfolgt nach den üblichen Verfahren der Peptidchemie. Diese Methoden sind ausführlich beschrieben in "Methoden der organischen Chemie" Band XV/1 und XV/2, Herausgeber: E. Müller, Georg-Thieme-Verlag, Stuttgart, 1974.

Als besonders zweckmäßig hat sich folgender Syntheseweg für die neuen Verbindungen erwiesen: Die Carbonsäure X-OH, in der eine gegebenenfalls vorhandene NH-Gruppe durch eine Benzyloxycarbonyl- oder Butyloxycarbonylgruppe geschützt ist, wird mit einem Aminosäureester der Formel II

$$H_2N\text{-}CR^2R^3\text{-}CC\text{-}OR^6 \quad (II),$$

worin $R^6$ eine Methyl- oder Ethylgruppe bedeutet, zu einem Zwischenprodukt der Formel III

$$X\text{-}NH\text{-}CR^2R^3\text{-}CO\text{-}OR^6 \quad (III),$$

umgesetzt. Hierzu ist es im allgemeinen notwendig, die freie Säurefunktion der Carbonsäure X-OH vor ihrer Einwirkung auf das

Aminosäurederivat II zu aktivieren. Aktivierte Derivate von Carbonsäuren sind bevorzugt die gemischten Anhydride, die in situ durch Einwirkung eines Chlorameisensäurealkylesters, wie beispielsweise Chlorameisensäureisobutylester oder Chlorameisensäureethylester dargestellt werden, Additionsprodukte an Carbodiimide - vorzugsweise Dicyclohexylcarbodiimid - oder aktivierte Ester, vorzugsweise die aus N-Hydroxysuccinimid und Dicyclohexylcarbodiimid gegebenenfalls in situ herstellbaren N-Hydroxysuccinimidester. Die Kondensation des aktivierten Derivats erfolgt in einem organischen Lösungsmittel, wie Dioxan, Tetrahydrofuran, Dichlormethan, Chloroform, Toluol oder Dimethylformamid oder in einem wäßrigorganischen Medium in Gegenwart einer Base. Als Base werden vorzugsweise Triethylamin, N-Methylmorpholin oder Natriumhydrogencarbonat verwendet. Die Reaktionstemperatur liegt zwischen -10 und +30° C, die Reaktionszeit beträgt 3 Stunden bis 4 Tage.

Die Estergruppe der Verbindungen III wird in Wasser oder in wäßrig-organischem Milieu durch Behandeln mit der äquivalenten Menge verdünntem Alkali bei 0 bis 40° C (Reaktionszeit 1 Stunde bis 2 Tage) hydrolytisch gespalten.

Nach ansäuern und entsprechender Aufarbeitung werden die Carbonsäuren IV erhalten:

$X\text{-}NH\text{-}CR^2R^3\text{-}CO\text{-}OH$ IV.

Die Carbonsäure IV wird mit einem Glycinderivat der Formel

$H_2N\text{-}CH_2\text{-}CO\text{-}R^1$ (V)

umgesetzt.

Dabei ist es im allgemeinen notwendig, die freie Säurefunktion der Carbonsäure IV vor der Einwirkung auf das Glycinderivat zu aktivieren. Diese Aktivierung geschieht in der bereits oben erwähnten Weise durch Bildung eines gemischten Säureanhydrids, eines Addukts an Carbodiimide oder eines aktivierten Esters.

Auf diese Weise erhält man die erfindungsgemäßen Verbindungen der Formel I, in denen X keinen Stickstoff enthält. Enthält X ein Stickstoffatom, so muß noch die Schutzgruppe abgespalten werden. Ist diese Benzyloxycarbonyl, erfolgt die Abspaltung zweckmäßig durch Hydrierung in Gegenwart eines Edelmetallkatalysators in einem inerten Lösungsmittel bei Raumtemperatur. Bevorzugte Edelmetallkatalysatoren sind Palladium, Platin oder Raney-Nickel. In der bevorzugten Ausführungsfotm wird beispielsweise 10 %iges Palladium auf Kohle verwendet. Als Lösungsmittel werden vorzugsweise Methanol, Essigsäureethylester oder Eisessig verwendet. Ist die Schutzgruppe t-Butyloxycarbonyl, erfolgt die Abspaltung zweckmäßig durch Behandeln mit einem Überschuß von Trifluoressigsäure oder mit einer Lösung von Chlorwasserstoff in einem inerten organischen Lösungsmittel wie Essigsäureethylester, Dioxan oder Tetrahydrofuran. Die Abspaltungsreaktion wird bei 0 bis 20° C durchgeführt. Die Reaktionsdauer beträgt 5 bis 30 Minuten.

Die neuen N-Acylderivate von Dipeptiden sind gegenüber der Einwirkung proteolytischer Enzyme weitgehend stabil, nach oraler Gabe wirksam und besitzen eine lange Wirkdauer.

Die Überlegenheit der neuen Substanzen zeigt sich insbesondere in folgenden Testmodellen:

1. Nach Everett, G.M. (in: Antidepressant Drugs, eds. Garattini, S. and Dukes, M.N.G., Amsterdam 1967, p. 164 ff.) wird eine Kombination von L-Dopa und Pargylin, die ein schwaches Erregungsbild hervorruft, Mäusen verabfolgt. Durch Vorbehandlung mit zentral erregenden Substanzen entwickelt sich erst ein ausgeprägtes Erregungsbild. Die neuen Tripeptide sind in diesem Testmodell bei oraler Verabfolgung in Dosierungen ab 0,02 mg/kg wirksam.

2. Die Applikationen des Cholinomimetikums Pilokarpin an der Ratte ruft neben peripheren Symptomen zentral Kratzen hervor (Kreiskott, H., Arch. exp. Path. Pharmak. 247, 317 (1964), das sich durch zentrale Anticholinergica, aber auch durch zentrale monoaminerge Stimulantien (Kreiskott, H. and Hofmann, H.P., 6. Int. Congress Pharmacol., Helsinki 1975, Abstr. 825) verhindern läßt. Orale Vorbehandlung mit den erfindungsgemäßen Tripeptiden unterdrückt das Pilokarpin-induzierte zentrale Kratzen.

3. Die in den Testmodellen 1 und 2 wirksamen Substanzen wurden zusätzlich an der Maus in einem Wirkungs- und Vergiftungsbild geprüft. Dabei werden die Symptome nach dem Verfahren von Irwin [Psychopharmakologia 13, 222 (1968)] erfaßt und quantifiziert. Die verschiedenen Testparameter werden kurz vor der Substanzgabe sowie 1/2, 1, 2, 3 und 24 Stunden nach oraler Gabe gemessen. Je Dosis werden Gruppen von 3 Tieren verwendet, die erste Verhaltensprüfung erfolgt nach 30 Minuten Eingewöhnung an den Makrolon-Käfig. Erfaßt werden Grundverhalten, zentrale Stimulation und Dämpfung sowie vegetative Symptome. Im einzelnen sind dies:

- Körperhaltung
- Stellung der Gliedmaßen
- Putzverhalten
- Stupor
- spontane und induzierte lokomotorische Aktivität
- Atmung
- sensomotorische Reaktionen (Reflexe)
- Lidspaltenweite
- Pupillengröße und
- Körpertemperatur u.a.

Die neuen Substanzen rufen eine Steigerung der Lokomotorik sowie verstärktes Schnüffeln, Aufrichten und Putzen hervor. Diese

Symptomatik tritt gleichermaßen bei dopaminerg wirksamen und Dopamin-stimulierenden Substanzen auf.

Die neuen Substanzen stimulieren demnach dopaminerge Abläufe deutlich.

Im Testmodell 1 (L-Dopa-Potenzierung) wird die Wirkung exogen zugeführten Dopamins gesteigert, in 2 (Pilokarpinerregung) und 3 (Wirkungsbild) wird das endogen vorhandene Dopamin in seinem Effekt verstärkt.

4. Mit mittleren Dosen Morphin subkutan vorbehandelte Ratten sind vom Gesamtverhalten her nicht auffällig. Kommt jedoch ein zusätzlicher äußerer Reiz hinzu, wie das Aufsetzen einer Klammer an den Schwanz, erstarren die Tiere schlagartig und zeigen Katalepsie. Nach Wegfall des Reizes sind die Ratten wieder unauffällig (Stille, G., Zur Pharmakologie katatonigener Stoffe, Aulendorf 1971, p. 30). Dieser reizinduzierte Zustand läßt sich durch intravenöse Injektion mit den beanspruchten Tripeptiden verhindern.

Die erfindungsgemäßen Peptide eignen sich daher allein oder in Kombination mit L-Dopa zur oralen Therapie des Morbus Parkinson und parkinsonoider Zustände sowie von Depressionen. Ferner können sie zur Verhinderung oder Behandlung der Opiatabhängigkeit eingesetzt werden.

Die erfindungsgemäßen Verbindungen können in üblicher Weise oral oder parenteral (subkutan, intravenös, intramuskulär, intraperitoneal) verabfolgt werden. Die Applikation kann auch mit Dämpfen oder Sprays durch den Nasen-Rachenraum erfolgen.

Die Dosierung hängt vom Alter, Zustand und Gewicht des Patienten sowie von der Applikationsart ab. In der Regel beträgt die tägliche Wirkstoffdosis zwischen etwa 0.1 und 100 mg/kg Körpergewicht bei oraler Gabe und zwischen etwa 0.01 und 10 mg/kg Körpergewicht bis parenteraler Gabe.

Die neuen Verbindungen können in den gebräuchlichen galenischen Applikationsformen fest oder flüssig angewendet werden, z. B. als Tabletten, Filmtabletten, Kapseln, Pulver, Granulate, Dragees, Suppositorien, Lösungen oder Sprays. Diese werden in üblicher Weise hergestellt. Die Wirkstoffe können dabei mit den üblichen galenischen Hilfsmitteln wie Tablettenbindern, Füllstoffen, Konservierungsmitteln, Tablettensprengmitteln, Fließreguliermitteln, Weichmachern, Netzmitteln, Dispergiermitteln, Emulgatoren, Lösungsmitteln, Retardierungsmitteln, Antioxidantien und/oder Treibgasen verarbeitet werden (vgl. H. Sucker et al: Pharmazeutische Technologie, Thieme-Verlag, Stuttgart, 1978). Die so erhaltenen Applikationsformen enthalten den Wirkstoff normalerweise in einer Menge von 0,1 bis 99 Gewichtsprozent.

Experimenteller Teil

Die folgenden Beispiele dienen zur Erläuterung der Erfindung, ohne sie zu beschränken.

Der Verlauf aller Reaktionen wurde mittels Dünnschichtchromatographie auf Kieselgelfertigplatten $F_{254}$ (Fa. Merck) verfolgt. Als Fließmittel wurden abhängig von der Polarität der untersuchten Verbindungen Dichlormethan/Aceton 20 : 1 bis 5 : 1, Dichlormethan/Methanol 20 : 1 bis 2 : 1 oder Butanol/Essigsäureethylester/Eisessig/Wasser 4 : 1 : 1 : 1 verwendet.

Die nach den folgenden Beispielen erhaltenen neuen Verbindungen sind dünnschichtchromatographisch rein.

Die NMR-Spektren sind im Einklang mit der angegebenen Struktur.

Celit® ist ein Filtrierhilfsmittel der Firma Johns-Manville.

Herstellung von D- bzw. L-2-Methylleucinmethylester

Zu einer Lösung von 233,0 g D,L-2-Methylleucinmethylester in 1170 ml Methanol wurden unter Rühren 220,0 g L(+)-Weinsäure zugegeben. Die Abscheidung des Tartrats von L-2-Methylleucimmethylester begann nach kurzer Zeit. Zur Vervollständigung der Fällung wurde über Nacht bei 5° C aufbewahrt. Der Niederschlag wurde abfiltriert, mit wenig Methanol gewaschen und zwischen 1500 ml Chloroform und 500 ml 10 %iger Soda-Lösung verteilt. Die wäßrige Phase wurde 2mal mit Chloroform extrahiert, die vereinigten Chloroformextrakte 3mal mit 100 ml Wasser gewaschen, über Natriumsulfat getrocknet und im Vakuum eingedampft. Ausbeute: 78 g L-2-Methylleucinmethylester (34 %), $[\alpha]^{20}_D = +20°C$ (c = 1,0 Dichlormethan).

Aus der Mutterlauge des Tartrats von L-2-Methylleucinmethylester wurden analog wie oben beschrieben die Base freigesetzt (130 g) und diese in Methanol mit 122 g D(-)-Weinsäure versetzt. Es fiel das Tartrat des D-2-Methylleucinmethylesters aus, das wie oben angegeben in D-2-Methylleucinnethylester (68 g, 31 %) überführt wurde, $[\alpha]^{20}_D = -21°$ (c = 1,0 Dichlormethan).

**Beispiel 1a**

Pyrrol-2-ylcarbonyl-L-2-methylleucyl-glycinamid

Zu 11,1 g Pyrrol-2-carbonsäure in 200 ml Dioxan wurden bei 10° C 23,0 g Dicyclohexylcarbodiimid und 16,0 g L-2-Methylleucinmethylester zugegeben. Das Reaktionsgemisch wurde 2 h bei 10° C und anschließend 24 h bei Raumtemperatur gerührt. Nach Filtration des entstandenen Dicyclohexylharnstoffs wurde im Vakuum eingedampft, der Rückstand in Essigsäureethylester aufgenommen und nacheinander mit 10 %iger wäßriger Zitronensäure-Lösung, Soda-Lösung und Wasser gewaschen, über Natriumsulfat getrocknet und im Vakuum eingedampft. Nach Reinigung durch Chromatographie an einer Kieselgelsäule (Elution

mit Dichlormethan/Essigsäureethylester 10 : 1) erhielt man 7,5 g Pyrrol-2-ylcarbonyl-L-2-methylleucinmethylester.

6,4 g Pyrrol-2-ylcarbonyl-L-2-methylleucimmethylester in 100 ml Dioxan/Wasser (4 : 1) wurden bei Raumtemperatur mit 13 ml 1 N Natriumhydroxid-Lösung versetzt. Nach 24 h wurde der Hauptteil des Dioxans im Vakuum abdestilliert, der Rückstand mit 50 ml Wasser versetzt und mehrmals mit Essigsäureethylester ausgezogen. Die wäßrige Phase wurde durch Zugabe von Zitronensäure auf pH 2 bis 3 gebracht und die entstandene Carbonsäure mit Essigsäureethylester extrahiert. Nach Waschen mit wenig Wasser, Trocknen über Natriumsulfat und Eindampfen im Vakuum erhielt man 6,0 g Pyrrol-2-ylcarbonyl-L-2-methylleucin.

Dieses wurde in 50 ml Dimethylformamid und 2,8 ml Triethylamin gelöst und bei -10° C unter Rühren tropfenweise mit 2,7 ml Chlorameisensäureisobutylester versetzt. Nach 15 min wurden zu der erhaltenen Lösung des asymmetrischen Säureanhydrids 3,3 g Glycinamid-Hydrochlorid in 10 ml Dimethylformamid und 4,2 ml Triethylamin zugegeben. Man rührte 1 h bei -10° C, dann 48 h bei Raumtemperatur und dampfte anschließend im Vakuum ein. Der Rückstand wurde in Essigsäureethylester aufgenommen, die organische Phase wurde nacheinander mit 10-%iger wäßriger Zitronensäure-Lösung, Soda-Lösung und Wasser gewaschen, über Natriumsulfat getrocknet und im Vakuum eingedampft. Chromatographie des Rückstands en einer Kieselgelsäule (Elution mit Dichlormethan/Methanol 5: 1) gab 3,3 g (56 %) Pyrrol-2-ylcarbonyl-L-2-methylleucin-glycinamid, Fp = 113 bis 128° C (Dichlormethan/Ether), $[\alpha]^{20}_D$ +3 (c = 0,5 Methanol).

Analog wurden erhalten:

1b. Pyrrol-2-ylcarbonyl-D-2-methylleucyl-glycinamid (48 %), Fp = 95 bis 125° C (Dichlormethan/Ether), $[\alpha]^{20}_D$ = -4° (c = 0,5 Methanol);

2a. Cyclopentyl-carbonyl-L-2-methylleucyl-glycinamid (59 %), Fp = 125 bis 131° C (Isopropanol/Ether), $[\alpha]^{20}_D$ = -12° (c = 0,5 Methanol);

2b. Cyclopentyl-carbonyl-D-2-methylleucyl-glycinamid (49 %), Fp = 124 bis 130° C (Isopropanol/Ether), $[\alpha]^{20}_D$ = +11° (c = 0,5 Methanol);

3a. Indol-2-ylcarbonyl-L-2-methylleucyl-glycinamid (37 %), Fp = 130 bis 140° C (Dichlormethan/Ether/Hexan), $[\alpha]^{20}_D$ = +11° (c = 0,5 Methanol);

3b. Indol-2-ylcarbonyl-D-2-methylleucyl-glycinamid (45 %), Fp = 120 bis 135° C (Dichlormethan/Ether/Hexan), $[\alpha]^{20}_D$ = -14° (c = 0,5 Methanol);

**Beispiel 4a**

L-Prolyl-L-2-methylleucyl-glycinamid

Zu 13,7 g N-Benzyloxycarbonyl-L-prolin und 7,7 ml Triethylamin in 90 ml Dimethylformamid wurden bei -10° C 7,3 ml Chlorameisensäureisobutylester zugetropft. Nach 15 min wurden zu der erhaltenen Lösung des asymmetrischen Säureanhydrids 8,8 g L-2-Methylleucinmethylester zugegeben. Man rührte 1 h bei -10° C, anschließend 16 h bei Raumtemperatur. Das Reaktionsgemisch wurde in Essigsäureethylester aufgenommen und die organische Phase nacheinander mit 10-%iger wäßriger Zitronensäure-Lösung, Soda-Lösung und Wasser gewaschen, über Natriumsulfat getrocknet und im Vakuum eingedampft. Ausbeute: 20,6 g N-Benzyloxycarbonyl-L-prolyl-L-2-methylleucinmethylester.

Dieses wurde in 250 ml Dioxan/Wasser (4: 1) bei Raumtemperatur gelöst und unter Rühren mit 55 ml 1 N Natriumhydroxid-Lösung in Portionen zu 1 ml unter Kontrolle des Alkaliverbrauchs mit Thymolphthalein als Indikator verseift. Nach Ansäuern mit der äquivalenten Menge Salzsäure wurde der Hauptteil des Dioxans im Vakuum abdestilliert. Man nahm in Essigsäureethylester auf und extrahierte mit verdünnter Kaliumhydrogencarbonat-Lösung. Nach ansäuern der wäßrigen Extrakte wurde mehrmals mit Essigsäureethylester extrahiert. Die vereinigten Extrakte wurden mit wenig Wasser gewaschen, über Natriumsulfat getrocknet und im Vakuum eingedampft. Ausbeute: 13,6 g N-Benzyloxycarbonyl-L-prolyl-L-2-methylleucin.

Zu 11,3 g N-Benzyloxycarbonyl-L-prolyl-L-2-methylleucin und 4,5 ml Triethylamin in 50 ml Dimethylformamid wurden bei -10° C unter Rühren 3,9 ml Chlorameisensäureisobutylester zugetropft. Nach 15 min bei -10° C wurden zu der erhaltenen Lösung des asymmetrischen Säureanhydrids 3,6 g Glycinamid-Hydrochlorid und 4,6 ml Triethylamin zugegeben. Man rührte 1 h bei -10° C und dann 48 h bei Raumtemperatur und dampfte anschließend im Vakuum ein. Der Rückstand wurde in Essigsäureethylester aufgenommen und die organische Phase nacheinander mit 10-%iger wäßriger Zitronensäure-Lösung, Soda-Lösung und Wasser gewaschen, über Natriumsulfat getrocknet und im Vakuum eingedampft. Der Rückstand wurde durch Chromatographie an einer Kieselgelsäure (Elution mit Dichlormethan/Methanol 5 : 1) gereinigt. Man erhielt 6,6 g N-Benzyloxycarbonyl-L-prolyl-L-2-methylleucyl-glycinamid.

4,6 g dieser Verbindung wurden in 150 ml Methanol gelöst und in Gegenwart von 0,5 g Palladium auf Kohle (10 %) hydriert. Nach Filtration über Celit® wurde im Vakuum eingedampft. Beim Anreiben mit Ether kristallisierten 2,7 g L-Prolyl-L-2-methylleucyl-glycinamid, Fp = 128 bis 132° C (Ether), $[\alpha]^{20}_D$ = -33° (c = 0,5 Methanol).

Analog wurden erhalten:

4b. L-Prolyl-D-2-methylleucyl-glycinamid (35

%), Fp = 155 bis 160° C (Ether/Hexan), $[\alpha]^{20}_D$ = -37° (c = 0,5 Methanol);

5a. L-Pyroglutamyl-L-2-methylleucyl-glycinamid (23 %), Fp = 188 bis 191° C (Methanol/Ether), $[\alpha]^{20}_D$ = -4° (c = 0,5 Methanol);

5b. L-Pyroglutamyl-D-2-methylleucyl-glycinamid (23 %), Fp = 202 bis 204° C (Dichlormethan/Ether), $[\alpha]^{20}_D$ = -25° (c = 0,5 Methanol);

**Beispiel 6a**

4,5-Dehydro-L-prolyl-L-2-methylleucyl-glycinamid

N-Butyloxycarbonyl-4,5-dehydro-L-prolin ergab in einer Beispiel 4a analogen Reaktionsfolge N-Butyloxycarbonyl-4,5-dehydro-L-prolyl-L-2-methylleucyl-glycinamid. Zur Abspaltung der Schutzgruppe wurde wie folgt verfahren: 0,8 g der Butyloxycarbonylverbindung wurden mit 15 ml 6 N Chlorwasserstoff in Dioxan 10 min bei Raumtemperatur behandelt. Anschließend wurde im Vakuum eingedampft, der Rückstand mehrmals mit Toluol coevaporiert und aus Isopropanol/Ether kristallisiert. Man erhielt 0,5 g 4,5-Dehydro-L-prolyl-L-2-methylleucyl-glycinamid, Fp = 137 bis 153° C (Isopropanol/Ether). Analog wurden hergestellt:

6b. 4,5-Dehydro-L-prolyl-D-2-methylleucyl-glycinamid (27 %), $[\alpha]^{20}_D$ = -15° (c = 0,5 Methanol);

7a. L-1,3-Thiazolidin-4-ylcarbonyl-L-2-methylleucyl-glycinamid-Hydrochlorid, Fp = 155 bis 165° C (Methanol/Ether), $[\alpha]^{20}_D$ = -61° (c = 0,5 Methanol);

7b. L-1,3-Thiazolidin-4-ylcarbonyl-D-2-methylleucyl-glycinamid-Hydrochlorid, Fp = 190 bis 200° C (Methanol/Ether), $[\alpha]^{20}_D$ = -50° (c = 0,5 Methanol);

8a. L-1,4-Thiazan-3-ylcarbonyl-L-2-methylleucyl-glycinamid-Hydrochlorid, Fp = 184 bis 188° C (Methanol/Ether), $[\alpha]^{20}_D$ +13° (c = 0,5 Methanol);

3b. L-1,4-Thiazan-3-ylcarbonyl-D-2-methylleucyl-glycinamid-Hydrochlorid, Fp = 176 bis 181° C (Methanol/Ether), $[\alpha]^{20}_D$ = +2° (c = 0,5 Methanol).

**Beispiel 9**

L-Prolyl-1-aminocyclopent-1-ylcarbonyl-glycinamid

Zu 5,6 g 1-Aminocyclopentan-1-carbonsäuremethylester in 50 ml Dioxan wurden bei 10° C 5,4 ml Triethylamin und 13,5 g des N-Hydroxysuccinimidesters von N-Benzyloxycarbonyl-L-prolin zugegeben. Das Reaktionsgemisch wurde 2 h bei 10° C gerührt, anschließend wurden 20 ml Wasser zugefügt und 20 h bei Raumtemperatur umgesetzt. Es wurde in Essigsäureethylester aufgenommen, die organische Phase nacheinander mit 10-%iger wäßriger Zitronensäure-Lösung, Soda-Lösung und Wasser gewaschen, über Natriumsulfat getrocknet und im Vakuum eingedampft. Kristallisation des festen Rückstandes gab 11,5 g (79 %) N-Benzyloxycarbonyl-L-prolyl-1-aminocyclopentan-1-carbonsäuremethylester.

8,2 g des so erhaltenen Methylesters wurden in 100 ml Methanol gelöst und nach Zugabe von 44 ml 1 N Natriumhydroxid-Lösung bei Raumtemperatur (20 h) verseift. Nach Ansäuern mit Salzsäure wurde im Vakuum zur Entfernung des Methanols eingeengt und die wäßrige Lösung mit Essigsäureethylester extrahiert. Die vereinigten Essigsäureethylesterextrakte wurden mit wenig Wasser gewaschen, über Natriumsulfat getrocknet und eingedampft. Der Rückstand wurde mehrmals mit Toluol coevaporiert. Ausbeute: 7,8 g (ca. 100 %) N-Benzyloxycarbonyl-L-prolyl-1-aminocyclopentan-1-carbonsäure.

Zu 7,7 g N-Benzyloxycarbonyl-L-prolyl-1-aminocyclopentan-1-carbonsäure und 2,9 ml Triethylamin in 30 ml Dimethylformamid wurden bei -10° C unter Rühren 2,7 ml Chlorameisensäureisobutylester zugetropft. Nach 15 min wurde zu der so erhaltenen Lösung des asymmetrischen Säureanhydrids eine Suspension von 2,3 g Glycinamid-Hydrochlorid in 20 ml Dimethylformamid und 2,9 ml Triethylamin zugegeben. Man rührte 1 h bei -10 bis 0° C und anschließend 48 h bei Raumtemperatur. Zur Aufarbeitung wurde in Essigsäureethylester aufgenommen und nacheinander mit 10-%iger wäßiger Zitronensäure-Lösung Soda-Lösung und Wasser gewaschen, über Natriumsulfat getrocknet und im Vakuum eingedampft.

Reinigung an Kieselgel (Elution mit Dichlormethan/Methanol 15 : 1) und anschließende Kristallisation aus Essigsäureethylester/Toluol gab 5,0 g (75 %) N-Benzyloxycarbonyl-L-prolyl-1-aminocyclopent-1-ylcarbonyl-glycinamid.

Dieses wurde in 100 ml Methanol gelöst und in Gegenwart von 0,5 g Palladium auf Kohle (10 %) hydriert. Nach Aufnahme der theoretischen Menge Wasserstoff wurde über Celit® filtriert, das Filtrat im Vakuum eingedampft und der Rückstand aus Isopropanol und Ether kristallisiert. Ausbeute: 2,9 g (86 %) L-Prolyl-1-aminocyclopent-1-ylcarbonyl-glycinamid, Fp = 167 bis 168° C (Isopropanol/Ether).

Analog wurden dargestellt:

10. L-Prolyl-1-aminocycloprop-1-ylcarbonyl-glycinamid (15 %), $[\alpha]^{20}_D$ = -37° (c = 0,5 Methanol);

11. L-Prolyl-1-aminocyclobut-1-ylcarbonyl-glycinamid (40 %), $[\alpha]^{20}_D$ = -39° (c = 0,5 Methanol);

12. L-Prolyl-1-aminocyclohex-1-ylcarbonyl-glycinamid (36 %), Fp = 143 bis 146° C (Isopropanol/Ether/Hexan), $[\alpha]^{20}_D$ = -36° (c = 0,6 Methanol);

13. L-Prolyl-1-aminocyclohept-1-ylcarbonyl-glycinamid (31 %), Fp = 170 bis 174°C (Isopropanol/Ether);

14. L-Prolyl-2-aminoindan-2-ylcarbonyl-glycinamid (29 %). Fp = 178 bis 181°C (Essigsäureethylester), $[\alpha]^{20}_D$ = -38° (c = 0,5 Methanol).

**Beispiel 15**

L-Prolyl-D-1-aminoindan-1-ylcarbonyl-glycinamid und L-Prolyl-L-1-aminoindan-1-ylcarbonyl-glycinamid

Zu 13,4 g l-Aminoindan-1-carbonsäuremethylester in 80 ml Dioxan wurden bei 10°C 11,4 ml Triethylamin und 26,0 g des N-Hydroxysuccinimidesters von N-Benzyloxycarbonyl-L-prolin zugegeben. Das Reaktionsgemisch wurde 4 h bei 10°C gerührt, anschließend wurden 15 ml Wasser zugefügt und 18 h bei Raumtemperatur umgesetzt. Es wurde in Essigsäureethylester aufgenommen und die organische Phase nacheinander mit 10-%iger wäßriger Zitronensäure-Lösung, Soda-Lösung und Wasser gewaschen, über Natriumsulfat getrocknet und im Vakuum eingedampft. Man erhielt 20,5 g (74 %) rohen Methylester der N-Benzyloxycarbonyl-L-prolyl-D,L-1-aminoindan-1-carbonsäure.

Dieser wurde in 300 ml Dioxan gelöst und nach Zugabe von 18,0 ml 4 N Natriumhydroxid-Lösung bei Raumtemperatur verseift. Nach Ansäuern mit verdünnter Salzsäure dampfte man das Dioxan im Vakuum weitgehend ab und nahm den Rückstand in Essigsäureethylester auf. Die organische Phase wurde mit Kaliumhydrogencarbonat-Lösung erschöpfend extrahiert. Nach Ansäuern der vereinigten wäßrigen Extrakte mit Salzsäure wurde mit Essigsäureethylester extrahiert. Man erhielt nach Trocknen der organischen Phase und Eindampfen im Vakuum 11,0 g (55 %) N-Benzyloxycarbonyl-L-prolyl-D,L-1-aminoindan -1-carbonsäure.

Zu 10,2 g N-Benzyloxycarbonyl-L-prolyl-D,L-1-aminoindan-1-carbonsäure und 3,8 ml Triethylamin in 30 ml Dimethylformamid wurden bei -10°C unter Rühren 3,5 ml Chlorameisensäureisobutylester zugetropft. Nach 10 min wurde zu dem so erhaltenen asymmetrischen Säureanhydrid eine Suspension von 3,0 g Glycinamid-Hydrochlorid in 30 ml Dimethylformamid und 3,8 ml Triethylamin zugegeben. Man rührte 1 h bei -10 bis 0°C und anschließend 48 h bei Raumtemperatur. Zur Aufarbeitung wurde in Essigsäureethylester aufgenommen und nacheinander mit 10-%iger wäßriger Zitronensäure-Lösung, Soda-Lösung und Wasser gewaschen, über Natriumsulfat getrocknet und im Vakuum eingedampft. Der Rückstand wurde an einer Kieselgelsäule (Elution mit Dichlormethan/Methanol 20 : 1) chromatographiert. In der Reihenfolge steigender Polarität wurden die beiden Isomeren in reiner Form erhalten:

N-Benzyloxycarbonyl-L-prolyl-D-1-aminoindan-1-ylcarbonyl-glycinamid: 3,9 g (34 %) und
N-Benzyloxycarbonyl-L-prolyl-L-1-aminoindan-1-ylcarbonyl-glycinamid: 0,8 g (7 %).

3,7 g N-Benzyloxycarbonyl-L-prolyl-D-1-aminoindan-1-ylcarbonyl-glycinamid wurden in 250 ml Methanol gelöst und in Gegenwart von 0,5 g Palladium auf Kohle (10 %) hydriert. Nach Aufnahme der theoretischen Menge Wasserstoff wurde über Celit® filtriert, das Filtrat im Vakuum eingedampft und aus Essigsäureethylester/Petrolether kristallisiert. Ausbeute: 2,1; (83 %) L-Prolyl-D-1-aminoindan-1-ylcarbonyl-glycinamid (15b), $[\alpha]^{20}_D$ = -86° (c = 0,5 Methanol).

Analog wurden aus 0,7 g N-Benzyloxycarbonyl-L-prolyl-L-1-aminoindan-1-ylcarbonyl-glycinamid 0,4 g (80 %) L-Prolyl-L-1-aminoindan-1-ylcarbonyl-glycinamid (15a) erhalten, $[\alpha]^{20}_D$ = +14° (c = 0,5 Methanol).

Analog wurden erhalten:

16a. L-Prolyl-L-2-amino-(2.2.1)-bicyclohept-2-ylcarbonyl-glycinamid (11 %), Fp = 233 bis 238°C (Dichlormethan/Petrolether), $[\alpha]^{20}_D$ = -55° (c = 0,5 Methanol); 16b. L-Prolyl-D-2-amino-(2.2.1)-bicyclohept-2-ylcarbonyl-glycinamid (26 %), Fp = 153 bis 160°C (Dichlormethan/Petrolether), $[\alpha]^{20}_D$ = +3° (c = 0,5 Methanol);

17a. L-Prolyl-L-2-methylphenylalanyl-glycinamid (20 %), Fp = 158 bis 164°C (Methanol/Ether/Hexan), $[\alpha]^{20}_D$ = +480 (c = 0 5 Methanol);

17b. L-Prolyl-D-2-methylphenylalanyl-glycinamid (18 %), Fp = 184 bis 186°C (Methanol/Ether/Hexan), $[\alpha]^{20}_D$ = -93° (c = 0,4 Methanol);

18a. L-Prolyl-L-2-amino-2-phenyl-propionyl-glycinamid (21 %), Fp = 138 bis 142°C (Dichlormethan/Ether/Hexan, $[\alpha]^{20}_D$ = -15° (c = 0,5 Methanol);

18b. L-Prolyl-D-2-amino-2-phenyl-propionyl-glycinamid (24 %), Fp = 86 bis 92°C (Dichlormethan/Ether/Hexan), $[\alpha]^{20}_D$ = -42° (c = 0,5 Methanol);

19a. L-Prolyl-L-2-amino-2-phenyl-butyryl-glycinamid (15 %), Fp = 151 bis 158°C (Isopropanol/Ether), $[\alpha]^{20}_D$ = -5° (c = 0,5 Methanol);

19b. L-Prolyl-D-2-amino-2-phenyl-butyryl-glycinamid (33 %), Fp = 174 bis 175°C (Isopropanol/Ether), $[\alpha]^{20}_D$ = -63° (c = 0,5 Methanol).

20a. L-Prolyl-L-2-amino-2-phenyl-valeryl-glycinamid (10 %), $[\alpha]^{20}_D$ = -4° (c = 0,5 Methanol).

20b. L-Prolyl-D-2-amino-2-phenyl-valeryl-glycinamid (30 %), Fp = 138 bis 141°C (Ether/Hexan), ($[\alpha]^{20}_D$ = -65° (c = 0,5 Methanol).

21a. L-Prolyl-L-2-amino-2,(1-naphthyl)-propionyl-glycinamid (10 %), Fp = 115 bis 120°C, $[\alpha]^{20}_D$ = -9° (c = 0,5 Methanol).

21b. L-Prolyl-D-2-amino-2-(1-naphthyl)-propionyl-glycinamid (25 %), Fp = 113 bis 120°C, $[\alpha]^{20}_D$ = -34° (c = 0,5 Methanol).

**Beispiel 22**

Analog Beispiel 15, aber mit der Änderung, daß die Benzyloxycarbonylschutzgruppen mit Bromwasserstoffsäure in Eisessig abgespalten wurden, erhielt man:

22a. L-Prolyl-L-2-amino-2-p-bromphenyl-butyryl-glycinamid-Hydrobromid (10 %), Fp = 196 bis 210° C, $[\alpha]^{20}_D$ = 0° (c = 0,5 Methanol) und

22b. L-Prolyl-D-2-amino-2-p-bromphenyl-butyryl-glycinamid-Hydrobromid (25 %), Fp = 185 bis 202° C, $[\alpha]^{20}_D$ = -51° (c = 0 5 Methanol).

Beispiele für galenische Zubereitungen:

**Beispeil A**

Auf einer Tablettenpresse werden in üblicher Weise Tabletten folgender Zusammensetzung gepreßt:

40 mg Substanz des Beispiels 2b
120 mg Maisstärke
13,5 mg Gelatine
45 mg Milchzucker
2,25 mg Aerosil® (chemisch reine Kieselsäure in submikroskopisch feiner Verteilung)
6,75 mg Kartoffelstärke (als 6-%iger Kleister)

**Beispiel B**

In üblicher Weise werden Dragees folgender Zusammensetzung hergestellt:

20 mg Substanz des Beispiels 2b
60 mg Kernmasse
60 mg Verzuckerungsmasse

Die Kernmasse besteht aus 9 Teilen Maisstärke, 3 Teilen Milchzucker und 1 Teil Luviskol[R] VA 64 (Vinylpyrrolidon-Vinylacetat-Mischpolymerisa 60 : 40, vgl. Pharm. Ind. 1962, 586). Die Verzuckerungsmasse besteht aus 5 Teilen Rohrzucker, 2 Teilen Maisstärke, 2 Teilen Calciumcarbonat und 1 Teil Talk. Die so hergestellten Dragees werden anschließend mit einem magensaftresistenten Überzug versehen.

**Patentansprüche** für die Vertragsstaaten BE, CH, DE, FR, GB, IT, LI, NL, SE.

1. N-Acylderivate von Peptiden der Formel I

$X-NH-CR^2R^3-CO-NH-CH_2-CO-R^1$ I,

in der

$R^1$ einen $C_{1-5}$-Alkoxyrest oder einen Aminorest der Formel $NR^4R^5$ darstellt worin $R^4$ und $R^5$ gleich oder verschieden sind und Wasserstoff oder $C_{1-5}$-Alkylreste bedeuten,

$R^2$ und $R^3$ gleich oder verschieden sind und $C_{1-5}$-Alkyl-reste oder Phenyl-, Benzyl- oder Naphthylreste oder zusammen mit dem ihnen gemeinsamen C-Atom auch ein aliphatisches Ringsystem mit 3 bis 7 Kohlenstoffatomen oder einen 1,1- oder 2,2-Indandiylrest bedeuten und

X einen Pyrrol-2-ylcarbonyl-, Indol-2-ylcarbonyl-, Cyclopentylcarbonyl-, L-Pyrrolidin-2-ylcarbonyl-, L-4,5-Dehydropyrrolidin-2-ylcarbonyl-, L-5-Oxopyrrolidin-2-ylcarbonyl-, L-1,3-Thiazolidin-4-ylcarbonyl- oder L-1,4-Thiazan-2-ylcarbonylrest bedeutet

sowie deren Salze mit physiologisch verträglichen Säuren.

2. Verfahren zur Herstellung von N-Acylderivaten von Peptiden der Formel I gemäß Anspruch 1, dadurch gekennzeichnet, daß man die Verbindungen

X-OH,
$H_2N-CR^2R^3-COOH$ und
$H_2N-CH_2-CO-R^1$

worin X, $R^1$, $R^2$ und $R^3$ die angegebene Bedeutung haben, nach in der Peptidchemie üblichen Verfahren miteinander kondensiert.

3. Arzneimittel enthaltend eine Verbindung der Formel I gemäß Anspruch 1.

4. Verbindung der Formel I gemäß Anspruch 1 zur Verwendung bei der Therapie von Krankheiten.

5. Verwendung der Verbindungen der Formel I gemäß Anspruch 1 zur Herstellung eines Arzneimittel zur Bekämpfung von Morbus Parkinson, parkinsonoider Zustände und Depressionen.

**Patentanspruch** für den Vertragsstaat AT.

Verfahren zur Herstellung von N-Acylderivaten von Peptiden der Fomel I

$X-NH-CR^2R^3-CO-NH-CH_2-CO-R^1$ I,

in der

$R^1$ einen $C_{1-5}$-Alkoxyrest oder einen Aminorest der Formel $NR^4R^5$ darstellt, worin $R^4$ und $R^5$ gleich oder verschieden sind und Wasserstoff oder $C_{1-5}$-Alkylreste bedeuten,

$R^2$ und $R^3$ gleich oder verschieden sind und $C_{1-5}$-Alkyl-reste oder Phenyl-, Benzyl- oder Naphthylreste oder zusammen mit dem ihnen gemeinsamen C-Atom auch ein aliphatisches Ringsystem mit 3 bis 7 Kohlenstoffatomen oder einen 1,1- oder 2,2-Indandiylrest bedeuten und

X einen Pyrrol-2-ylcarbonyl-, Indol-2-ylcarbonyl-, Cyclopentylcarbonyl-, L-Pyrrolidin-2-ylcarbonyl-, L-4,5-Dehydropyrrolidin-2-ylcarbonyl-,L-5-Oxopyrrolidin-2-ylcarbonyl-, L-1,3-Thiazolidin-4-ylcarbonyl- oder L-1,4-Thiazan-2-ylcarbonylrest bedeutet .

sowie deren Salzen mit physiologisch verträglichen Säuren, dadurch gekennzeichnet, daß man die Verbindungen

X-OH,
$H_2N-CR^2R^3-COOH$ und
$H_2N-CH_2-CO-R^1$

worin X, $R^1$, $R^2$ und $R^3$ die angegebene Bedeutung haben, nach in der Peptidchemie

üblichen Verfahren miteinander kondensiert.

**Claims** for the Contracting States BE, CH, DE, FR, GB, IT, LI, NL, SE.

1. An N-acyl derivative of a peptide of the formula I

$X-NH-CR^2R^3-CO-NH-CH_2-CO-R^1$ I,

where

$R^1$ is $C_1$-$C_5$-alkoxy or an amino radical of the formula $NR^4R^5$, where $R^4$ and $R^5$ are identical or different and are each hydrogen or $C_1$-$C_5$-alkyl,

$R^2$ and $R^3$ are identical or different and are each $C_1$-$C_5$-alkyl, phenyl, benzyl or naphthyl or, together with the carbon atom which they share, may furthermore form an aliphatic ring system of 3 to 7 carbon atoms or 1,1- or 2,2-indanediyl radical, and

X is pyrrol-2-ylcarbonyl, indol-2-ylcarbonyl, cyclopentylcarbonyl, L-pyrrolidin-2-ylcarbonyl, L-4,5-dehydropyrrolidin-2-ylcarbonyl, L-5-oxopyrrolidin-2-ylcarbonyl, L-1,3-thiazolidin-4-ylcarbonyl or L-1,4-thiazan-2-yl-carbonyl,

and its salts with physiologically tolerated acids.

2. A process for the preparation of an N-acyl derivative of a peptide of the formula I as claimed in claim 1, wherein the compounds

X-OH,

$H_2N-CR^2R^3-COOH$, and

$H_2N-CH_2CO-R^1$

where X, $R^1$, $R^2$ and $R^3$ have the specified meanings, are condensed with one another by a method conventionally used in peptide chemistry.

3. A drug containing a compound of the formula I as claimed in claim 1.

4. A compound of the formula I as claimed in claim 1 for use in the treatment of disorders.

5. The use of a compound of the formula I as claimed in claim 1 for the preparation of a drug for the therapy of Parkinson's disease, Parkinson-like conditions and depressions.

**Claim** for the Contracting State AT

A process for the preparation of an N-acyl derivative of a peptide of the formula I

$X-NH-CR^2R^3-CO-NH-CH_2-CO-R^1$ I,

where

$R^1$ is $C_1$-$C_5$-alkoxy or an amino radical of the formula $NR^4R^5$, where $R^4$ and $R^5$ are identical or different and are each hydrogen or $C_1$-$C_5$-alkyl,

$R^2$ and $R^3$ are identical or different and are each $C_1$-$C_5$-alkyl, phenyl, benzyl or naphthyl or, together with the carbon atom which they share, may furthermore form an aliphatic ring system of 3 to 7 carbon atoms or a 1,1- or 2,2-indanediyl radical, and

X is pyrrol-2-ylcarbonyl, indol-2-ylcarbonyl, cyclopentylcarbonyl, L-pyrrolidin-2-ylcarbonyl, L-4, 5-dehydropyrrolidin-2-ylcarbonyl, L-5-oxopyrrolidin-2-ylcarbonyl, L-1,3-thiazolidin-4-ylcarbonyl or L-1,4-thiazan-2-yl-carbonyl,

and its salts with physiologically tolerated acids, wherein the compounds

X-OH,

$H_2N-CR^2R^3-COOH$, and

$H_2N-CH_2-CO-R^1$

where X, $R^1$, $R^2$ and $R^3$ have the specified meanings, are condensed with one another by a method conventionally used in peptide chemistry.

**Revendications** pour les Etats contractants BE, CH, DE, FR, BG, IT, LI, NL, SE.

1. Dérivés N-acylés de peptides de la formule I

$X - NH - CR^2R^3 - CO - NH - CH_2 - CO - R^1$ (I),

dans laquelle

$R^1$ désigne un radical alcoxy en $C_1$ à $C_5$ ou un groupe amino de la formule $NR^4R^5$, où $R^4$ et $R^5$, qui peuvent être identiques ou différents, désignent chacun un atome d'hydrogène ou un radical alkyle en $C_1$ à $C_5$,

$R^2$ et $R^3$, qui peuvent être identiques ou différents, désignent chacun un radical alkyle en $C_1$ à $C_5$ ou un groupe phényle, benzyle ou naphtyle, ou forment avec l'atome de carbone commun un système cyclique aliphatique comprenant trois à sept atomes de carbone ou un groupe indane-diyle-1,1 ou -2,2 et

X peut être choisi parmi les groupes pyrrole-2-yl-carbonyle, indole-2-yl-carbonyle, cyclopentylcarbonyle, L-pyrrolidine-2-yl-carbonyle, L-4,5-déhydropyrrolidine-2-yl-carbonyle, L-5-oxopyrrolidine-2-yl-carbonyle, L-1,3-thiazolidine-4-yl-carbonyle et L-1,4-thiazane-2-yl-carbonyle,

ainsi que les sels d'acides physiologiquement compatibles de ces dérivés.

2. Procédé de préparation de dérivés N-acylés de peptides de la formule I suivant la revendication 1, caractérisé en ce que l'on soumet à une réaction de condensation, selon l'une des techniques connues dans le domaine de la chimie des peptides, des composés des formules

X - OH,

$H_2N - CR^2R^3 - COOH$

et $H_2N - CH_2 - CO - R^1$,

dans lesquelles X, $R^1$, $R^2$ et $R^3$ possèdent la signification définie.

3. Médicament, contenant un composé de la formule I suivant la revendication 1.

4. Composé de la formule I suivant la revendication 1, destiné au traitement thérapeutique de maladies.

5. Utilisation de composés de la formule I suivant la revendication 1 pour la préparation d'un médicament destiné au traitement de la maladie de Parkinson, d'états parkinsonoïdes et de dépressions.

**Revendication** pour l'Etat contractant AT

Procedé de préparation de dérivés N-acylés de peptides de la formule I

X - NH - $CR^2R^3$ - CO - NH - $CH_2$ - CO - $R^1$ (I),

dans laquelle

$R^1$ désigne un radical alcoxy en $C_1$ à $C_5$ ou un groupe amino de la formule $NR^4R^5$, où $R^4$ et $R^5$ qui peuvent être identiques ou différents, désignent chacun un atome d'hydrogène ou un radical alkyle en $C_1$ à $C_5$,

$R^2$ et $R^3$, qui peuvent être identiques ou différents, désignent chacun un radical alkyle en $C_1$ à $C_5$ ou un groupe phényle, benzyle ou naphtyle, ou forment avec l'atome de carbone commun un système cyclique aliphatique comprenant trois à sept atomes de carbone ou un groupe indane-diyle-1,1 ou -2,2 et

X peut être choisi parmi les groupes pyrrole-2-ylcarbonyle, indole-2-yl-carbonyle, cyclopentylcarbonyle, L-pyrrolidine-2-yl-carbonyle, L-4,5-déhydropyrrolidine-2-yl-carbonyle, L-5-oxopyrrolidine-2-yl-carbonyle, L-1,3-thiazolidine-4-yl-carbonyle et L-1,4-thiazane-2-yl-carbonyle,

ainsi que de sels d'acides physiologiquement compatibles de ces dérivés, caractérisé en ce que l'on soumet à une réaction de condensation, selon l'une des techniques connues dans le domaine de la chimie des peptides, des composés des formules

X - OH,

$H_2N$ - $CR^2R^3$ - COOH

et $H_2N$ - $CH_2$ - CO - $R^1$,

dans lesquelles X, $R^1$, $R^2$ et $R^3$ possèdent la signification définie.